# EUROPEAN PATENT APPLICATION

(11) **EP 4 116 406 A1**
(43) Date of publication of application: **11.01.2023**
(21) Application number: 22155449.6
(22) Date of filing: 07.02.2022
(51) Int. Cl.: C12N 1/00, C12N 5/00, C12P 23/00

(54) **METHOD FOR CULTURING HAEMATOCOCCUS PLUVIALIS CONTAINING LARGE AMOUNT OF ASTAXANTHIN**

(30) Priority: 07.07.2021 KR 20210088867; 25.08.2021 KR 20210112040
(71) Applicant: Korea University Research and Business Foundation, Seoul 02841 (KR)
(72) Inventor: Sim, Sang Jun, Seoul (KR); Sung, Young Joon, Seoul (KR); Cho, Seung Jun, Anyang-si (KR)
(74) Representative: Schön, Christoph

(57) **Abstract**

Disclosed is a method for culturing *Haematococcus pluvialis* containing a large amount of astaxanthin. According to one embodiment, the method includes culturing *Haematococcus pluvialis* under autotrophic conditions to prepare a culture containing cysts in which astaxanthin is accumulated and adding a nitrogen source to the culture to induce germination of the cysts.

## Description

### BACKGROUND OF THE INVENTION

### 1. Field of the Invention

The present invention relates to a method for culturing *Haematococcus pluvialis* containing a large amount of astaxanthin, and more specifically to a technology for biomass production in which the inherent germination characteristics of the microalga *Haematococcus pluvialis* are used to enhance the productivities of biomass and astaxanthin from *Haematococcus pluvialis.*

### 2. Description of the Related Art

Astaxanthin is a pigment that belongs to the xanthophyll family of carotenoids. Astaxanthin has attracted considerable attention in medical applications for its functions such as antioxidant, anticancer, and anti-inflammatory activities. Astaxanthin is divided into natural astaxanthin and synthetic astaxanthin. Particularly, synthetic astaxanthin accounts for more than 95% of the market supply because it can be produced at low cost. However, synthetic astaxanthin has several problems in terms of stability and continuous supply because it is obtained from petroleum resources. Thus, numerous studies have focused on the synthesis of natural astaxanthin. *Haematococcus pluvialis,* a unicellular green alga, accumulates astaxanthin to up to 4% of its dry cell weight. For this reason, *Haematococcus pluvialis* has been spotlighted as a source for the production of naturally occurring astaxanthin. Technologies for the economical production of astaxanthin through photosynthesis can be combined with carbon capture, utilization, and storage technologies. For example, some studies have been conducted on the production of astaxanthin from *Haematococcus pluvialis* using flue gas from power plants as a carbon source and sunlight as an energy source. However, this approach for astaxanthin production suffers from problems such as fungal contamination, low physiological activity of astaxanthin at high temperature, and slow growth of *Haematococcus pluvialis,* limiting its commercial use.

### SUMMARY OF THE INVENTION

The present invention has been made in an effort to solve the above-described problems and an aspect of the present invention is to provide a method for culturing *Haematococcus pluvialis* capable of effectively producing astaxanthin by applying the cyst germination of *Haematococcus pluvialis* to a semi-continuous process.

A method for culturing *Haematococcus pluvialis* according to one embodiment of the present invention includes (a) culturing *Haematococcus pluvialis* under autotrophic conditions to prepare a culture containing cysts in which astaxanthin is accumulated and (b) adding a nitrogen source to the culture to induce germination of the cysts.

According to one embodiment of the present invention, the initial concentration of the cells in the culture before the induction of germination in step (b) may be 0.7 to 1.7 g/L.

According to one embodiment of the present invention, light with an intensity of 200 to 400 µE/m²/s may be irradiated in step (b).

According to one embodiment of the present invention, in step (b), the cysts may germinate into zooids and the zooids may be converted back into cysts where a large amount of astaxanthin is accumulated when the culture is depleted of the nitrogen source.

According to one embodiment of the present invention, step (b) may be carried out in a semi-continuous process.

According to one embodiment of the present invention, step (b) may include (b1) harvesting some of the cells from the culture in a state in which the culture is depleted of the nitrogen source after the induction of germination and (b2) further adding the nitrogen source to the remaining culture to reinduce germination and substeps (b1) and (b2) may be sequentially repeated.

According to one embodiment of the present invention, the method may be carried out in an outdoor photobioreactor to which LNG-fired flue gas is supplied.

The features and advantages of the present invention will become apparent from the following description with reference to the accompanying drawings.

Prior to the detailed description of the invention, it should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

According to the present invention, the cyst germination of *Haematococcus pluvialis* is applied to a semi-continuous process so that *Haematococcus pluvialis* containing a large amount of astaxanthin can be produced on a large scale under autotrophic conditions in an economical and simple manner, creating a high added value. Specifically, the present invention eliminates the problems of low productivity and inefficiency encountered in conventional batch culture and overcomes the limitations of conventional semi-continuous processes where the use of reactors and space utilization are inefficient and light intensity conditions vary depending on cell growth and astaxanthin accumulation. Therefore, the present invention is expected to contribute to the commercial utilization of biomass produced from *Haematococcus pluvialis.*

In addition, the present invention enables the mass production of biomass in a short period of time. Therefore, the present invention can be used to significantly reduce carbon dioxide emissions with high efficiency when combined with technologies for reducing carbon dioxide emissions.

### BRIEF DESCRIPTION OF THE DRAWINGS

These and/or other aspects and advantages of the invention will become apparent and more readily appreciated from the following description of the embodiments, taken in conjunction with the accompanying drawings of which:
Fig. 1 is a flow diagram schematically showing a method for culturing *Haematococcus pluvialis* according to one embodiment of the present invention;
Fig. 2 shows images showing changes in cell morphology during culture of *Haematococcus pluvialis* according to one embodiment of the present invention;
Fig. 3 shows a process for enhancing astaxanthin production by a method for culturing *Haematococcus pluvialis* according to one embodiment of the present invention;
Fig. 4 shows changes in the number of cells after germination at various initial biomass *(Haematococcus pluvialis)* concentrations;
Fig. 5 shows (a) changes in the concentration of residual nitrogen and (b) changes in reactive oxygen species (ROS) level after germination at various initial biomass *(Haematococcus pluvialis)* concentrations;
Fig. 6 shows (a) changes in cell density and (b) changes in astaxanthin content after germination at various initial biomass *(Haematococcus pluvialis)* concentrations;
Fig. 7 shows (a) changes in astaxanthin concentration and (b) changes in the cumulative amount of astaxanthin produced in a semi-continuous process and a batch process;
Fig. 8 shows the expression levels of four genes, *psy, crtO, bkt2,* and *crtR-B,* in *Haematococcus pluvialis* (a) when cultured in a semi-continuous process and (b) when cultured in a batch process; and
Fig. 9 shows (a) changes in cell density and (b) changes in astaxanthin content when a semi-continuous process and a batch process were separately applied to large-scale outdoor culture systems using flue gas.

### DETAILED DESCRIPTION OF THE INVENTION

The objects, specific advantages, and novel features of the present invention will become more apparent from the following detailed description and preferred embodiments, examples of which are illustrated in the accompanying drawings. In the description of the present invention, detailed explanations of related art are omitted when it is deemed that they may unnecessarily obscure the essence of the present invention.

Preferred embodiments of the present invention will now be described in detail with reference to the accompanying drawings.

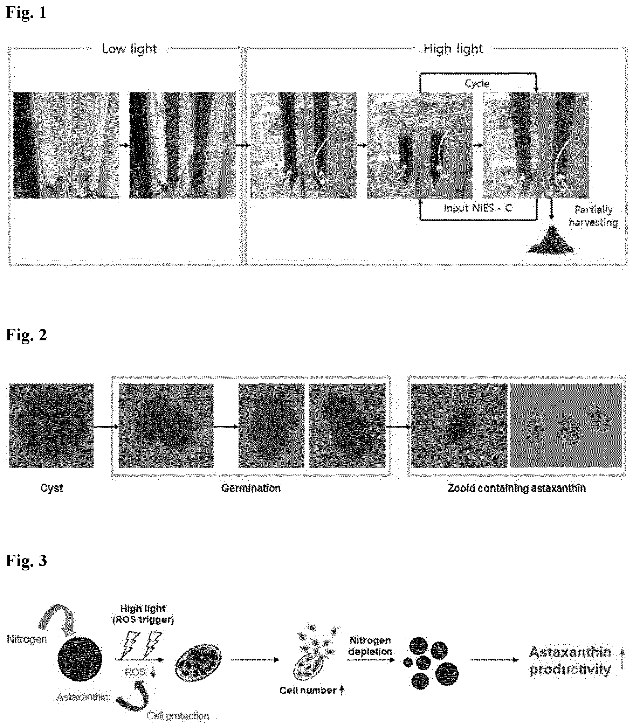

Fig. 1 is a flow diagram schematically showing a method for culturing *Haematococcus pluvialis* according to one embodiment of the present invention, Fig. 2 shows images showing changes in cell morphology during culture of *Haematococcus pluvialis* according to one embodiment of the present invention, and Fig. 3 shows a process for enhancing astaxanthin production by a method for culturing *Haematococcus pluvialis* according to one embodiment of the present invention.

As shown in Figs. 1 to 3, the method includes culturing *Haematococcus pluvialis* under autotrophic conditions to prepare a culture containing cysts in which astaxanthin is accumulated and adding a nitrogen source to the culture to induce germination of the cysts.

The present invention is directed to a method for culturing *Haematococcus pluvialis* containing a large amount of astaxanthin.

*Haematococcus pluvialis* is a microalgal species that grows in freshwater and has the ability to accumulate astaxanthin, a carotenoid, to 3 to 4% of its dry weight. *Haematococcus pluvialis* forms cysts to resist various stresses such as nutrient depletion, exposure to strong light, increased salinity, and very low or high temperatures. In this course, *Haematococcus pluvialis* accumulates secondary metabolites such as lipids and carotenoids to resist stresses. Particularly, astaxanthin (3,3'-dihydroxy-β,β'-carotene-4,4'-dione), a red ketocarotenoid, has attracted considerable attention in medical applications for its effects such as antioxidant, anticancer, and anti-inflammatory activities. *Haematococcus pluvialis* is a microalgal species capable of producing astaxanthin and undergoes two different stages: a growth stage and a cyst stage for astaxanthin accumulation. The cell wall surrounding *Haematococcus pluvialis* cells is composed of 70% carbohydrates (66% hexose), 3% celluloses, 6% proteins, and 3% acetolysis-resistant materials. Astaxanthin accumulates in the thick cell wall of *Haematococcus pluvialis* cysts.

Technologies for the economical production of astaxanthin through photosynthesis can be combined with carbon capture, utilization, and storage technologies. For example, some studies have been conducted on the production of astaxanthin from *Haematococcus pluvialis* using flue gas from power plants as a carbon source and sunlight as an energy source. However, this approach for astaxanthin production suffers from problems such as fungal contamination, low physiological activity of astaxanthin at high temperature, and slow growth of *Haematococcus pluvialis,* limiting its commercial use.

Microalgal culture conditions are divided into autotrophic, heterotrophic, and mixotrophic conditions. Microalgal culture under autotrophic conditions uses fixed carbon dioxide as a carbon source without the supply of any organic carbon source and light energy to synthesize organic compounds. Autotrophic conditions are more suitable for long-term culture than heterotrophic and mixotrophic conditions because they are safer from causative bacteria and fungi of contamination. However, despite its ability to produce biomass containing a large amount of astaxanthin, culture under autotrophic conditions is limited in that the culture period is long.

Biological processes offer only limited CO₂ emission reductions per unit area because of their characteristics. Due to this limitation, improved carbon dioxide reduction capacities of microalgae-based biomass production processes are required for facilities where large amounts of greenhouse gases are emitted and a large amount of carbon dioxide should be disposed of.

Thus, the present invention intends to provide a solution to the problems of conventional culture methods for *Haematococcus pluvialis.*

As described above, the method for culturing *Haematococcus pluvialis* according to the present invention includes (S100) preparing a culture and (S200) inducing germination (S200).

In step (S100), *Haematococcus pluvialis* is cultured to prepare a culture containing culturing *Haematococcus pluvialis* cells. The culture of *Haematococcus pluvialis* is performed under autotrophic conditions and the cells present in the culture are cysts in which astaxanthin is accumulated.

The culture may be prepared in a batch process. *Haematococcus pluvialis* may be inoculated into a medium in a reactor and light is irradiated thereto with supply of carbon dioxide to form cysts. *Haematococcus pluvialis* undergoes growth and induction stages during culture to form cysts in which astaxanthin is accumulated. Weak light with an intensity of 40 to 80 µE/m²/s may be irradiated in the growth stage and strong light with an intensity of 200 to 400 µE/m²/s may be irradiated in the induction stage.

In step (S200), a nitrogen source is added to the culture to induce germination of the cysts. The cells present in the culture are cultured and converted into cysts in the absence of a nitrogen source. The addition of the nitrogen source causes cell division inside the cysts and makes the cell wall surrounding the cysts thinner, eventually leading to release of a large number of zooids through the cell wall. As a result, the number of cells increases. The cysts formed in a culture environment without any nitrogen source rapidly consume the added nitrogen source until the culture becomes depleted of the nitrogen source. Here, when the nitrogen source is depleted to create a favorable condition for astaxanthin production, the zooids are converted back into cysts. The zooids have a size of 10 to 20 µm in diameter while the cysts grow to a size of 20 to 50 µm. Thus, the larger cysts produce more biomass than the zooids. Overall, according to the method for biomass production through cyst germination, strong light irradiated can be effectively used, the number of cells can be effectively increased simply by the supply of a nitrogen source, and rapid nitrogen source depletion allows zooids to be converted into cysts where biomass production can be effectively enhanced.

The initial concentration of the cells and the intensity of light are important for effective astaxanthin production. The initial concentration of the cells in the culture before the induction of germination (that is, the cells for germination) is preferably 0.7 to 1.7 g/L. It is also preferable to irradiate light with an intensity of 200 to 400 µE/m²/s. A detailed description of the initial concentration of the cells and the intensity of light will be given with reference to experimental examples.

In step (S200), germination may be induced in a semi-continuous process. As an example, step (S200) may include (S210) harvesting some of the cells and (S220) reinducing germination and substeps (S210) and (S220) may be sequentially repeated. Specifically, in substep (S210), some of the cells are harvested from the culture in a state in which the culture is depleted of the nitrogen source after the induction of germination. In substep (S220), the nitrogen source is further added to the remaining culture to reinduce germination. In substep (S220), it is preferable that the initial concentration of the cells to be germinated is maintained at 0.7 to 1.7 g/L and the light intensity is 200 to 400 µE/m²/s. By sequentially repeating substeps (S210) and (S220) several times, large quantities of biomass and astaxanthin can be produced.

The method for culturing *Haematococcus pluvialis* may be carried out in an outdoor photobioreactor to which LNG-fired flue gas is supplied.

The present invention will be explained in more detail with reference to the following experimental examples.

### 1. Materials and Methods

### 1.1. Strain and culture conditions

In this experiment, cells of *Haematococcus pluvialis* strain NIES-144 obtained from the National Institute for Environmental Studies (NIES), Japan were cultured in NIES-C medium. The pH was maintained at a constant level using HEPES serving as a buffer. 30 mL of the culture was introduced into a 100 mL flask, followed by culture in a 5% CO₂ incubator (Vision Scientific, South Korea) at 25 °C. Light with an intensity of 50 µEm⁻²s⁻¹ was irradiated in the growth stage.

### 1.2. Culture in batch process and photoautotrophic induction

The cells in the growth stage were inoculated into 100 mL of NIES-C medium in a 250 mL flask and cultured in a 5% CO₂ incubator at 130 rpm and 25 °C. Two-stage culture was performed in a batch process to enhance photoautotrophic astaxanthin production. The cells in the growth stage were cultured in NIES-C. Light with an intensity of 50 µEm⁻²s⁻¹ was irradiated in the growth stage and its intensity was changed to 250 µEm⁻²s⁻¹ in the induction stage for astaxanthin production. The growth and induction stages in the batch process lasted for 8 and 20 days, respectively.

### 1.3. Culture in semi-continuous process

For culture in a semi-continuous process, 100 mL of NIES-C medium was introduced into a 250 mL flask and culture was done in a 5% CO₂ incubator at 130 rpm and 25 °C.

Red cysts were centrifuged in the induction stage and resuspended in NIES-C. At that time, light with an intensity of 250 µEm⁻²s⁻¹ was irradiated. Cells were cultured at various initial biomass concentrations of 0.3, 1.2, 2.1, and 3.0 gL⁻¹.

### 1.4. Large-scale cultivation in photobioreactor as outdoor culture system

Flue gas from a liquefied natural gas (LNG) plant was used as a CO₂ source. The flue gas was composed of CO₂ (3-6%), O₂ (11.99 ± 0.73%), CO (1.43 ± 4.03 ppm), and NOₓ (21.72 ± 3.72 ppm). The flue gas was supplied at a flow rate of 0.1 vvm to a photobioreactor (PBR, Photobioreactor) as an outdoor culture system. For the batch process, cells grown in the growth stage were inoculated at a biomass concentration of 0.03 gL⁻¹, light with an intensity of 50 µEm⁻²s⁻¹ was irradiated in the growth stage, and light with an intensity of 250 µEm⁻²s⁻¹ was irradiated in the induction stage. For the semi-continuous process, light with an intensity of 250 µEm⁻²s⁻¹ was irradiated. The mean temperature over the entire experimental period was 22 ± 4 °C.

### 1.5. Nitrate concentrations and cell numbers

Cells in the germination stage were observed with a microscope (Olympus CKX41, Japan) and their numbers (cells/mL) were measured with a hemocytometer (Incyto, South Korea). Nitrate concentrations were determined with a Spectroquant^{®} test kit (Merck, 1.14773.0001).

### 1.6. Determination of reactive oxygen species levels

Intracellular reactive oxygen species (ROS) levels were measured using 2',7'-dichlorodihydrofluorescein diacetate (H₂DCFDA) according to the previously reported method (Shim, S.J, Hong, M.E, Chang, W.S, Sim, S.J, 2020. Repeated-batch production of omega-3 enriched biomass of Chlorella sorokiniana via calcium-induced homeoviscous adaptation. Bioresour. Technol. 303, 122944.). Cells were centrifuged and resuspended in an extraction buffer containing 5 µM H₂DCFDA. The cell suspension was incubated at 25 °C for 10 min and protected from light. The cells thus treated were centrifuged and washed twice with the extraction buffer. Thereafter, the cell suspension was homogenized and dichlorofluorescein (DCF) was extracted. The resulting sample was centrifuged and the level of reactive oxygen species level in the supernatant was measured with a Tecan multireader.

### 1.7. RNA extraction and real-time qPCR

In order to find the cause of the rapid increase in the content of astaxanthin in the semi-continuous process compared to in the batch process, real-time qPCR analysis was conducted to analyze the relative expression levels of genes involved in astaxanthin biosynthesis.

### 1.8. Dry weight analysis

Biomass dry weights were measured using GF/F glass microfiber filters. After each sample was passed through the microfiber filter, the filter attached with biomass was dried at 75 °C. The difference in the weight of the filter before and after the treatment was calculated and determined as a biomass dry weight.

### 1.9. Astaxanthin extraction and analysis

The cell suspension was centrifuged and astaxanthin was extracted with a Tissue Lyser II (Qiagen, USA) using glass beads and methanol. After saponification with a 10 mM KOH solution, the astaxanthin content was analyzed by HPLC.

### 2. Results and Discussion

### 2.1. Effect of cyst germination on cell number

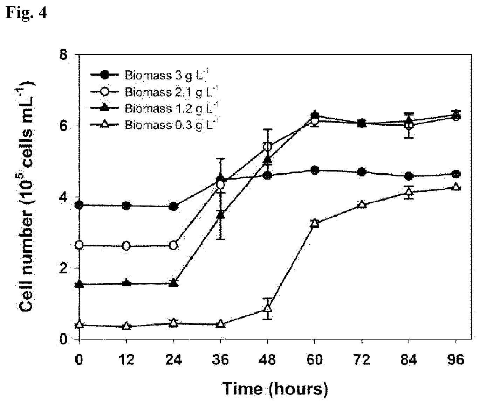

Cyst germination of *Haematococcus pluvialis* is primarily dependent on nitrogen concentration and light intensity. A nitrogen source was added at different initial biomass concentrations of 0.3, 1.2, 2.1, 3.0 gL⁻¹ to induce cyst germination and changes in the number of *Haematococcus pluvialis* cells depending on the initial biomass concentrations were analyzed. The results are shown in Fig. 4. Fig. 4 shows changes in the number of cells after germination at various initial biomass (*Haematococcus pluvialis*) concentrations.

Since the same amount of the nitrogen source was introduced into each sample, it was expected that the increase in the number of cells would be greatest at the lowest initial biomass concentration. Referring to Fig. 4, the largest increase in cell number (962%) was observed at the lowest initial biomass concentration (0.3 gL⁻¹) (314% at 1.2 gL⁻¹, 137% at 2.1 gL⁻¹, and 23% at 3.0 gL⁻¹). In the samples other than the sample at the lowest biomass concentration (0.3 gL⁻¹), no changes in cell number were initially observed and the cell number began to increase after 24 h. It took twice as long for the cell number to start to increase at the lowest biomass concentration compared to at the other biomass concentrations. Since the self-shading effect is minimized at a low cell concentration, cells may be immediately exposed to strong light and reactive oxygen species (ROS)-related stress conditions, resulting in slow response to external stimuli and inhibition of cell division.

Cysts were converted into zooids during germination, and 60 h later, most of the zooids are converted back into cysts to protect themselves from oxidative stress, except at the lowest biomass concentration (0.3 gL⁻¹). This is because cysts are more advantageously protected from light than zooids due to their lower sensitivity to light.

60 h later, the conversions into cysts (the proportions of cysts in total cells) at initial biomass concentrations of 3.0, 2.1, and 1.2 gL⁻¹ were 100, 98.6, and 92.6%, respectively.

The increased number of cells after germination and the rapid conversion of zooids into cysts are responsible for effective astaxanthin accumulation in cells.

### 2.2. Nitrogen consumption and reactive oxygen species formation during germination

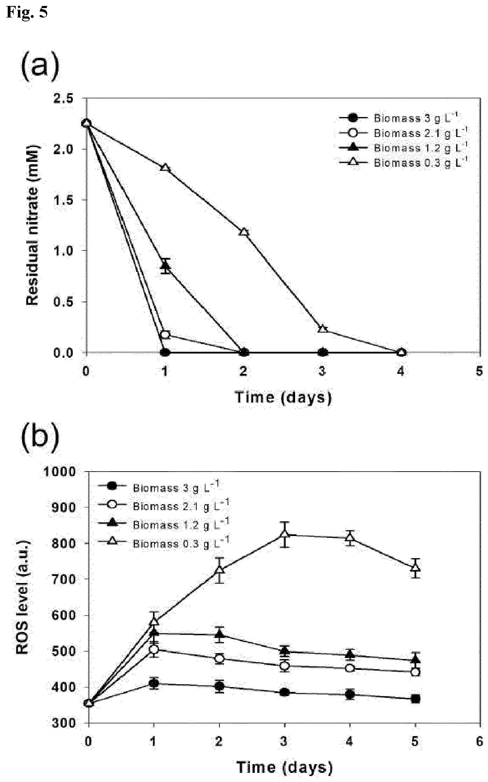

Various concentrations of mature red cysts were inoculated into fresh NIES-C media supplemented with 2.2 mM nitrate. Since nitrogen is an important nutrient for cell growth and germination, cellular physiological changes can be inferred by analyzing the concentration of residual nitrogen in culture medium. Fig. 5 shows (a) changes in the concentration of residual nitrogen and (b) changes in reactive oxygen species (ROS) level after germination at various initial biomass (*Haematococcus pluvialis*) concentrations.

As shown in (a) of Fig. 5, the nitrogen consumption rate varied depending on the initial biomass concentration. That is, the higher the initial biomass concentration, the faster the nitrogen was depleted. Nitrogen was depleted within 2 days at initial biomass concentrations of 2.1 and 1.2 gL⁻¹ and within one day at an initial biomass concentration of 3 gL⁻¹. On day 4, no nitrogen was left in the culture media at all concentrations.

After the cell number was rapidly increased by cyst germination, the depletion of the nitrogen source enables efficient astaxanthin accumulation in cells. When ROS are generated by light with a high intensity, oxidative stress causes cell damage. *Haematococcus pluvialis* cells produce astaxanthin that effectively resists oxidative stress to inhibit the release of ROS.

When the initial number of cells is small, a relatively large amount of nitrogen is available to each cell. The addition of nitrogen sources restores cellular metabolism but protective pigments, including astaxanthin, can absorb light to reduce photosynthetic energy production. Thus, the degradation of astaxanthin is promoted and the depletion of a nitrogen source important for astaxanthin synthesis is delayed for efficient use of light energy under culture conditions where the initial number of cells is small. As a result, when cells are cultured at a low concentration, a low concentration of astaxanthin does not effectively inhibit ROS induced by strong light, resulting in an increase in ROS level. This result is confirmed by an increased ROS level at a low cell concentration (see (b) of Fig. 5).

### 2.3. Comparison of biomass concentrations and astaxanthin contents during germination

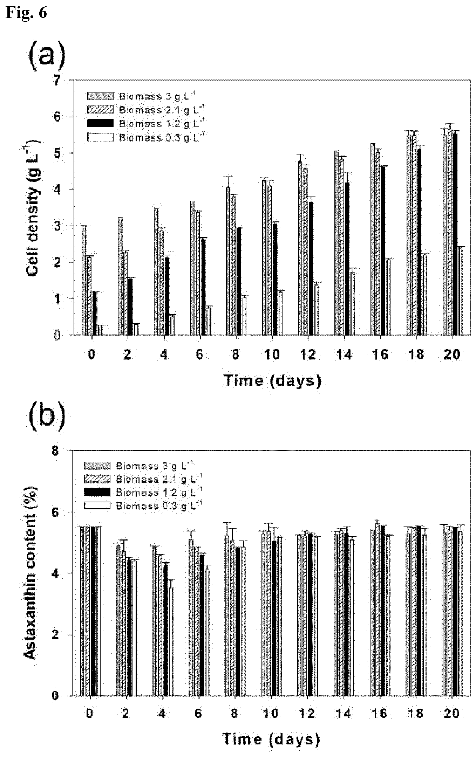

After germination induction by introduction of the nitrogen source, cell densities and astaxanthin contents were measured. The results are shown in Fig. 6. Fig. 6 shows (a) changes in cell density and (b) changes in astaxanthin content after germination at various initial biomass (*Haematococcus pluvialis*) concentrations.

Referring to (a) of Fig. 6, the cell numbers were increased during germination, leading to continuous increases in cell density. However, excessive exposure of cells to oxidative stress induced by strong light inhibited the growth of cysts. As a result, at a low biomass concentration (0.3 gL⁻¹), individual cells were significantly exposed to strong light inducing excessive oxidative stress, leading to an inefficient increase in cell density.

In contrast, at high initial cell concentrations, limited biomass increases were observed due to the relatively low nitrogen concentrations. Biomass productivities of 0.12, 0.17, 0.21, and 0.11 gL⁻¹d⁻¹ were measured at initial biomass concentrations of 3, 2.1, 1.2, and 0.3 gL⁻¹, respectively. That is, the most effective increase in cell density was achieved at an initial biomass concentration of 1.2 gL⁻¹. Nitrogen inhibits the expression of genes involved in astaxanthin synthesis and induces germination. Strong light is one of the most important factors for astaxanthin production. Therefore, strong light needs to be irradiated onto the culture in order to minimize a decrease in astaxanthin accumulation caused by the supply of the nitrogen source.

Referring to (b) of Fig. 6, the astaxanthin contents steadily decreased for 4 days of germination and thereafter they began to increase. The highest astaxanthin productivity was 11.7 mgL⁻¹d⁻¹ at an initial biomass concentration of 1.2 gL⁻¹. In conclusion, nitrogen-mediated germination at an initial biomass concentration of 1.2 gL⁻¹ in the semi-continuous process can maximize astaxanthin production.

### 2.4. Comparison of amounts of astaxanthin produced in batch process and semi-continuous process

The market value of astaxanthin produced by *Haematococcus pluvialis* has been on the rise. Under such circumstances, various types of semi-continuous processes have been developed to enhance biomass productivity. According to conventional semi-continuous processes, fresh culture media are supplied to highly physiologically active cells in the growth stage to minimize the lag phase, resulting in improved cell growth.

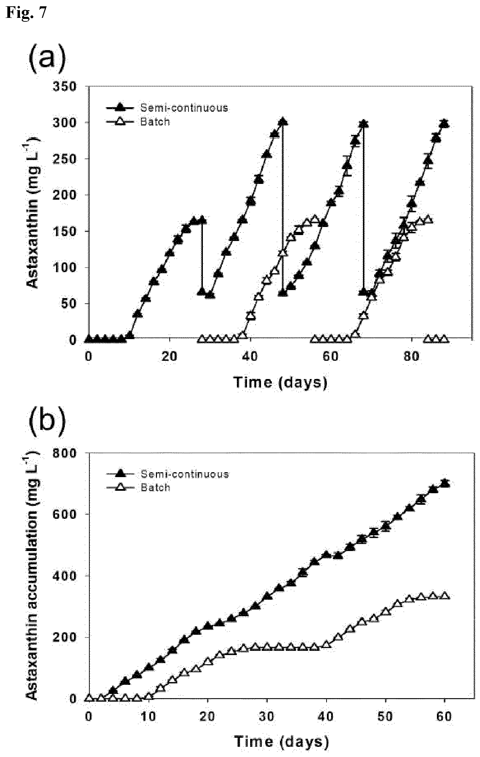

However, other conventional semi-continuous processes where culture conditions and cell states are completely different are inefficient in producing astaxanthin from *Haematococcus pluvialis* cells. In contrast, according to the method of the present invention, germination, which is a unique biological phenomenon of *Haematococcus pluvialis,* is applied to a semi-continuous process for efficient astaxanthin production. In order to verify the effect of the present invention, astaxanthin productivity in a semi-continuous process where optimal germination induction conditions were applied (at an initial biomass concentration of 1.2 gL⁻¹) was compared with that in a batch process. Fig. 7 shows (a) changes in astaxanthin concentration and (b) changes in the cumulative amount of astaxanthin produced in the semi-continuous process and the batch process.

Referring to (a) of Fig. 7, mature red cysts obtained in the batch process were used in the first cycle of the semi-continuous process. As a result, a higher astaxanthin concentration could be maintained in the semi-continuous process compared to in the batch process and no decrease in astaxanthin content was found when the culture cycle was repeated.

After the first batch process, the amount of astaxanthin accumulated in the semi-continuous process was compared with that in the batch process. Referring to (b) of Fig. 7, 700.4 and 331.7 mgL⁻¹ of astaxanthin were produced for 60 days in the semi-continuous process and the batch process, respectively. Astaxanthin could not be continuously accumulated in the batch process because the growth of vegetative cells was required to increase the number of cells. In contrast, the number of astaxanthin-containing cells was increased by germination in the semi-continuous process, resulting in an increase in astaxanthin production.

In conclusion, the semi-continuous process based on germination can increase the astaxanthin productivity by a factor of 2.1 times compared to the conventional batch process.

### 2.5. Transcript expression of carotenoid biosynthetic genes during germination

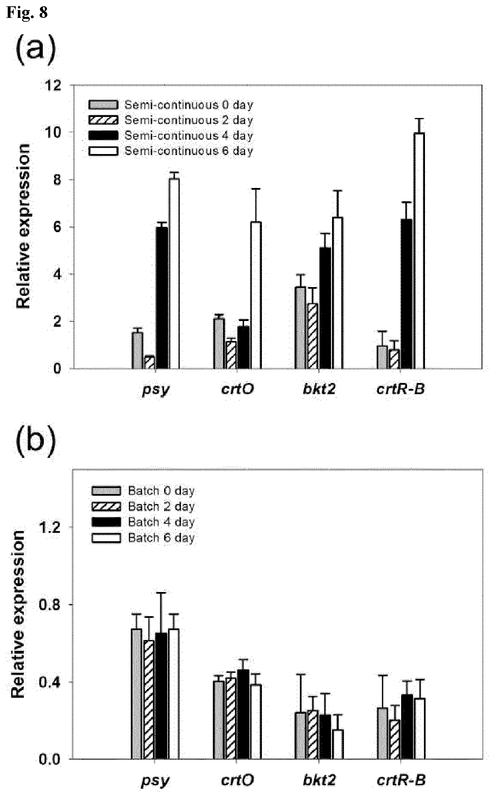

The expression levels of four genes, *psy, crtO, bkt2,* and *crtR-B,* related to carotene biosynthesis in the semi-continuous and batch processes were measured over 0, 2, 4 and 6 days by real-time qPCR. Phytoene synthase (*psy*) is a key enzyme in astaxanthin biosynthesis. *crtO* and *bkt2* are β-carotene ketolases/oxygenases that promote astaxanthin biosynthesis by the action of *crtR-B.* The expression level of *crtR-*B is known to have a significant correlation with astaxanthin. Fig. 8 shows the expression levels of four genes, *psy, crtO, bkt2,* and *crtR-B,* in *Haematococcus pluvialis* (a) when cultured in the semi-continuous process and (b) when cultured in the batch process.

Time-course analysis revealed that these four genes showed the same expression profile in the semi-continuous process (see (a) of Fig. 8). The expression levels of the genes were reduced for 2 days after nitrogen addition, during which the residual nitrogen source was consumed as a nutrient, resulting in inhibition of carotenoid biosynthesis. When the cells were irradiated with strong light after nitrogen depletion, the expression levels of all carotenoid-related genes increased continuously. These results concluded that light with a high intensity increases the expression levels of genes, *psy, crtO, bkt2,* and *crtR-B.*

In contrast, the expression levels of the carotenoid-related genes were maintained low and astaxanthin was hardly accumulated in the batch process due to the growth stage where the number of cells increased (see (b) of Fig. 8).

In conclusion, nitrogen addition for cyst germination and high light intensity enhance the expression levels of the genes, contributing to effective astaxanthin production after cyst germination.

### 2.6. Large-scale outdoor production of astaxanthin using flue gas

Based on the foregoing experimental examples, both the semi-continuous process and the batch process were carried out in outdoor systems using flue gas from liquefied natural gas (LNG) plants. Here, photobioreactors (PBR) made of polymer films were used.

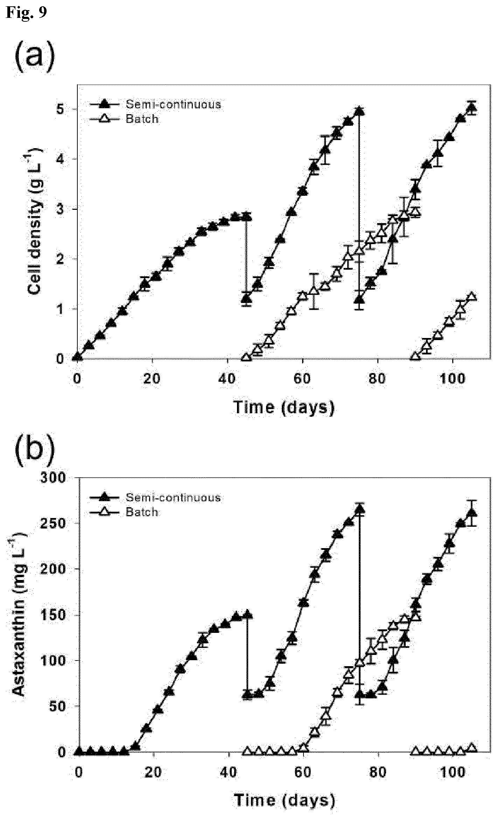

The semi-continuous process based on germination was carried out every 30 days identical to the period of the astaxanthin induction stage in the batch process. Fig. 9 shows (a) changes in cell density and (b) changes in astaxanthin content when the semi-continuous process and the batch process were separately applied to large-scale outdoor culture systems using flue gas.

The photobioreactors for outdoor culture are bulky compared to those for laboratory culture, making it difficult to efficiently transmit light to individual cells. Further, since complex temperature control systems were not used taking into consideration operating costs and energy consumption, biomass and astaxanthin productivities were rather low in outdoor culture compared to in laboratory culture.

However, the biomass (see (a) of Fig. 9) and astaxanthin productivities (see (b) of Fig. 9) were increased by 1.8 and 2.6 times, respectively, when the semi-continuous process based on germination was applied to the outdoor system using flue gas compared to when the batch process was applied. The semi-continuous process was successfully repeated in a large-scale photobioreactor.

Since the culture cycle of the semi-continuous process is shorter than that of the batch process, more energy is needed to harvest cells and introduce media and cells. Nevertheless, since the market price of astaxanthin is very high, the increased astaxanthin content of cells can offset the disadvantage of the additional energy input. In addition, since the downstream process for extracting astaxanthin accumulated in *Haematococcus pluvialis* cells accounts for the largest portion of the overall production operation cost, a high astaxanthin content can save the operating cost when the same amount of biomass is treated.

In conclusion, the semi-continuous process requires an additional energy input and an increased operating cost but is advantageous in terms of overall economic efficiency due to its enhanced astaxanthin productivity.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the present invention belong to the scope of the present invention, and the specific scope of the present invention will be clearly defined by the appended claims.

## Claims

1. A method for culturing *Haematococcus pluvialis* comprising (a) culturing *Haematococcus pluvialis* under autotrophic conditions to prepare a culture containing cysts in which astaxanthin is accumulated and (b) adding a nitrogen source to the culture to induce germination of the cysts.

2. The method according to claim 1, wherein the initial concentration of the cells in the culture before the induction of germination in step (b) is 0.7 to 1.7 g/L.

3. The method according to claim 1, wherein light with an intensity of 200 to 400 µE/m²/s is irradiated in step (b).

4. The method according to claim 1, wherein, in step (b), the cysts germinate into zooids and the zooids are converted back into cysts where a large amount of astaxanthin is accumulated when the culture is depleted of the nitrogen source.

5. The method according to claim 1, wherein, step (b) is carried out in a semi-continuous process.

6. The method according to claim 5, wherein, step (b) comprises (b1) harvesting some of the cells from the culture in a state in which the culture is depleted of the nitrogen source after the induction of germination and (b2) further adding the nitrogen source to the remaining culture to reinduce germination and wherein substeps (b1) and (b2) are sequentially repeated.

7. The method according to claim 1, wherein the method is carried out in an outdoor photobioreactor to which LNG-fired flue gas is supplied.
